# EUROPÄISCHE PATENTSCHRIFT

(11) **EP 0 890 100 B1**
(45) Veröffentlichungstag und Bekanntmachung des Hinweises auf die Patenterteilung: **08.09.2004**
(21) Anmeldenummer: 97906970.5
(22) Anmeldetag: 24.03.1997
(51) Int. Cl.: G01N 33/00, H01J 49/04

(54) **ANORDNUNG ZUM VERBINDEN EINES TIEFDRUCKEINGANGES EINES GASANALYSEGERÄTES**
ARRANGEMENT FOR CONNECTING A LOW-PRESSURE INLET OF A GAS ANALYSER
DISPOSITIF POUR LE RACCORDEMENT D'UNE ENTREE BASSE PRESSION DANS UN APPAREIL D'ANALYSE DE GAZ

(30) Priorität: 27.03.1996 DE 29605650 U
(43) Veröffentlichungstag der Anmeldung: 13.01.1999
(73) Patentinhaber: Unaxis Balzers Aktiengesellschaft, 9496 Balzers (LI)
(72) Erfinder: RETTINGHAUS, Gerhard, FL-9496 Balzers (LI)
(74) Vertreter: Troesch Scheidegger Werner AG
(86) Internationale Anmeldenummer: PCT/CH1997/000122
(87) Internationale Veröffentlichungsnummer: WO 1997/036177

(56) Entgegenhaltungen:
- EP-A- 0 550 957
- DE-A- 2 033 954
- DE-A- 2 939 893
- US-A- 3 633 027
- US-A- 5 318 752

## Beschreibung

Die vorliegende Erfindung betrifft eine Anordnung nach dem Oberbegriff von Anspruch 1, ein Massenspektrometer mit einer solchen und eine Verwendung der erwähnten Anordnung und des Massenspektrometers.

Massenspektrometer als typisches Beispiel der erwähnten Gasanalysegeräte werden verbreitet eingesetzt, z.B. um Gaszusammensetzungen zu analysieren. Insbesondere bei Vakuumbehandlungsprozessen, wie bei PVD-, PECVD- oder CVD-Verfahren, ist es mit zunehmendem Masse erforderlich, insbesondere für kritische Anwendungen, die Behandlungsgasatmosphäre genau zu beherrschen. Dabei werden CVD-Verfahren auch unter atmosphärischen Druckbedingungen eingesetzt.

Die Gasanalysegeräte arbeiten üblicherweise in Vakuumbereichen, die wesentlich tiefer sind, beispielsweise im 10⁻³mbar-Bereich, als die Prozessdrucke, welche bei den erwähnten Verfahren eingesetzt werden, von beispielsweise 0,1mbar bis 100mbar. Die damit einhergehenden Probleme bei der Ankopplung der Gasanalysegeräte der genannten Art an die zu analysierenden Testgäsatmosphären sind bekannt: Verfälschung der Gaszusammensetzung bei der Ueberführung des Gases zum Analysegerät, Verfälschung der Gaszusammensetzung im Analysegerät selbst bzw. Beschädigung oder mindestens Verunreinigung des Analysegerätes durch zu untersuchendes Gas.

Diese Probleme sind dann verschärft, wenn es sich bei dem Testgas um ein Reaktivgas handelt, welches mit Feststoffphasen im Verbindungssystem und/oder am Gasanalysegerät reagieren kann, oder wenn ein solches Reaktivgas feste Reaktionsprodukte bildet, die sich im genannten Verbindungssystem und/oder eingangsseitig des Gasanalysegerätes niederlegen.

Es sind verschiedene Vorgehen bekannt, diese Probleme zu lösen.

Eine erste bekannte Vorgehensweise ist es, eine Druckstufe in der Verbindungsleitung vorzusehen, in Form einer Blende. Das Vorsehen einer solchen fixen Druckstufe, wie beispielsweise aus der US-A-5 318 752 bekannt, hat den Nachteil, dass damit der höchstmögliche Betriebsdruck des Gasanalysegerätes oft nicht ausgenutzt werden kann. Bei wechselnden Drucken des zu analysierenden Gases wäre deshalb eine Variationsmöglichkeit der Blende bzw. Druckstufe wünschbar. Nur damit könnte in jedem Fall ein optimales Verhältnis zwischen Nutzsignal und Untergrundsignal am Analysegerät sichergestellt werden.

Dies wird durch das ebenfalls bekannte vorsehen eines einstellbaren Leckventils als Druckstufe gewährleistet. Vorsehen eines Ventilmechanismus im erwähnten Verbindungssystem bringt aber schwerwiegende Nachteile mit sich, mit Bezug auf Vergrösserung der gasabsorbierenden Oberflächen, Einführen zusätzlicher Totvolumina mit entsprechenden Memory-Effekten sowie bezüglich der Verunreinigung der Gasatmosphäre, beispielsweise durch mechanischen Abrieb.

Die Nachteile der erwähnten mechanischen Leckventile wurden teilweise durch die Einführung der sogenannten "virtual valves" gelöst, ohne aber dabei die Vorteile der mechanischen Ventile gänzlich beizubehalten. Es handelt sich bei den "virtual valves" um gasdynamische Druckstufen bzw. Abschlüsse, bei denen dem Analysegasfluss zum Analysegerät hin, im Gegenstrom, eine Sperrgasströmung entgegengesetzt wird. Bei entsprechender Sperrgasströmung kann das Analysegas vom Testeingang des Analysegerätes weitgehendst ferngehalten werden. Auch diesbezüglich kann auf die US-A-5 318 752 verwiesen werden.

Der wesentlichste Nachteil der "virtual valves" ist es, dass die Sperrgasströmung es nicht erlaubt, einen vollständigen Verschluss, wie er beispielsweise bei der Belüftung einer Prozesskammer, woran das Analysegerät angeschlossen ist, notwendig ist, vorzunehmen. Zudem ergibt sich an der Einmündung des Gaseinlasses für das Sperrgas in das Verbindungssystem eine Stelle, woran Memory-Effekte auftreten können. Dies, indem beispielsweise am erwähnten Gaseinlass alte Gasreste adsorbiert bleiben, die später, bei abgestelltem Sperrgas, desorbiert werden und folgende Analysen verfälschen.

Aus der US 3 633 027 ist ein sogenannter Becker-Ryhage-Separator bekannt, an welchem ein Pumpanschluss über eine Eingangsdüse auf den Eingang eines Gaschromatographen geführt ist. Gegenüber einer Eingangsdüse zum Gaschromatographen ist eine Ausgangsdüse zu einer zweiten, einer Massenspektrometerstufe, vorgesehen. Ein in zwei diskrete Positionen verstellbarer Schieber ist in einem Führungsgehäuse geführt und verschliesst oder öffnet die erwähnten Düsen.

Aus der DE-OS 29 39 893 ist es weiter bekannt, ein Massenspektrometer über eine Verbindungsleitung mit zwei Anschlüssen zu beaufschlagen, welche Verbindungsleitung einen dritten Anschluss für das Massenspektrometer aufweist. Ein im Impulsbetrieb betriebener Ventilkörper steuert dabei den Strömungsquerschnitt von der Verbindungsleitung zum Massenspektrometer.

Es ist Aufgabe der vorliegenden Erfindung, die vorbeschriebenen Nachteile bezüglich Memory-Effekten an einer Anordnung letztgenannter Art zu beheben. Dies wird durch ihre Ausbildung nach dem Kennzeichen von Anspruch 1 erreicht.

Durch Vorsehen eines Leckventils und damit variable Ausbildung der Druckstufe - Einstellung mittels des Leckventilkörpers - wird auch eine vollständige Abtrennung des Analysegeräteeinganges möglich. Dadurch, dass weiter der Gasströmungsanschluss an eine Leitung angeschlossen ist, die zwischen Ventilkörper und dessen Führung gebildet ist, wird dieses Zwischenvolumen, welches ansonsten zum Totvolumen würde, optimal genutzt, sei dies, um mittels einer Sperrgasströmung eine "virtual valve" zu realisieren oder grundsätzlich, um die Vorteile einer Gegengasströmung zur Analysegasströmung auszunützen, sei dies, um durch differentielles Abpumpen durch diese Leitung das Entstehen von Memory-Effekten im erwähnten Volumen zu verhindern.

Die Erfindung wird anschliessend beispielsweise anhand einer Figur erläutert.

Diese zeigt, schematisch, eine erfindungsgemässe Verbindungsanordnung bzw. ein erfindungsgemässes Massenspektrometer, vorzugsweise Quadrupolmassenspektrometer, mit einer erfindungsgemässen Verbindungsanordnung.

### Definitionen:

| | |
|---|---|
| Testgas Testgaseingang Testgasatmosphäre Testkammer | Zu analysierendes Gas wird über einen Eingang aus einer Gasatmosphäre entnommen. Letztere herrscht in einer Kammer. Bei der Atmosphäre kann es sich vorzugsweise um eine Vakuumatmosphäre handeln, dabei z.B. um die Prozessatmosphäre eines Vakuumbehandlungsprozesses. |
| | |
| Ventil Ventilkörper Ventilantrieb Ventilkörperführung | Ein Ventil weist den gesteuert in einer Ventilkörperführung beweglichen Ventilkörper auf. Der Ventilkörper wird darin durch den Ventilantrieb angetrieben. |

An einer Verbindungsleitung 1, üblicherweise als "baffle"-Leitung bezeichnet, ist, einerseits, ein Testgaseingang 3, beispielsweise in Form eines Flansches z.B. zum Anschliessen an eine Testkammer 5, beispielsweise eine Vakuumbehandlungskammer, wie z.B. einer Aetzkammer, vorgesehen. Ein Leckventil 7 mit Antriebseinheit 9 verschliesst, gesteuert, als variable Druckstufe 13, den Strömungsquerschnitt der Verbindungsleitung 1 zum Eingang E eines Gasanalysegerätes 15, wie vorzugsweise eines Quadrupolmassenspektrometers mit angeschlossener Turbomolekularpumpe 17. Der Ventilkörper 11 ist in einer Ventilkörperführung 19 beweglich und definiert bezüglich der Führung 19 die Leitung 21. Diese ist über einen Anschluss 23 und ein Stellventil 24 an einen Sperrgastank 26 angeschlossen sowie, über einen Anschluss 28 und ein Stellventil 30, an eine Vakuumpumpe 32.

Im Betrieb wird je nach Druckverhältnissen in der Testgasatmosphäre A die Druckstufe 13 durch mehr oder weniger weites Rückholen des Ventilkörpers 11 eingestellt. Gleichzeitig können, durch Erstellen einer Gegengasströmung S über Anschluss 23, die bekannten Vorteile einer Gegengasströmung bezüglich Partialdruckverhältnissen eingangsseitig des Gasanalysegerätes ausgenützt werden. Hierzu kann, wie gestrichelt bei 34 dargestellt ist, ein zusätzlicher Pumpanschluss zwischen Gaseinlass aus Leitung 21 und Testgaseingang 3 vorgesehen werden. Die Gasgegenströmung S verhindert, dass Testgas im Bereich des Volumens der Leitung 21 und insbesondere auch in deren Einmündungsbereich in Leitung 1 absorbiert wird.

Andernfalls kann auch über den Anschluss 28 eine Testgasströmung Aₙ durch die Leitung 21 erstellt werden, welche jegliche Rückströmung aus der Leitung 21 verhindert.

In Sperrperioden, in welchen eine vollständige Absperrung des Einganges E nicht gefordert ist, wird durch eine entsprechend bemessene Sperrgasströmung S Durchdringen von Testgas zum Eingang E des Massenspektrometers 15 verhindert. Das "virtual valve" ist dann geschlossen. Bei notwendiger vollständiger Absperrung, wie zum Fluten der Atmosphäre A, wird das Leckventil mit Ventilkörper 11 geschlossen.

Mit dem erfindungsgemässen Verbindungssystem kann der Druck im Messgerät dem Druck in der Testgasatmosphäre A angepasst werden, nämlich durch entsprechende Einstellung des Strömungsquerschnittes mittels des Leckventils 7, ohne dass die sonst üblichen Nachteile eines Leckventils in Kauf zu nehmen wären. Unabhängig von wechselnden Testgasdrucken erhält man damit immer den bestmöglichen Abstand zwischen Testgas-Partialdruck und Restgas-Partialdruck.

Durch Vorsehen des Leckventils 7 ist es möglich, den hochempfindlichen Eingang E des Gasanalysegerätes 15 gar gegen Umgebungsatmosphärendruck abzusperren. Auch wenn dabei der Ventilkörper 11 eine vollständige Schliessung des Strömungsquerschnittes 13 nicht absolut sicherstellt, ergibt sich immer noch die Möglichkeit, durch gleichzeitiges Erstellen einer Sperrgasströmung S den Eingang E optimal zu schützen.

Durch Variation des Strömungsquerschnittes entsprechend Druckstufe 13 werden Abschätzungen des Eigenuntergrundes am Analysegerät 15 möglich, z.B., bei einem Massenspektrometer, seiner Ionenquelle. Memory- und Sicker-Effekte aus dem Leckventilvolumen werden durch dessen Ausnützung als Sperrgasleitung und/oder als Pumpleitung vermieden. Die bekannten Vorteile von Gegenstrom-Messverfahren können flexibel ausgenützt werden; die Testgasströmung durch Leitung 1 kann durch Ausnützung der Leitung 21 als Pumpleitung erhöht werden.

Bilden sich in der Druckstufe Ablagerungen, die den Leitwert vermindern, so kann der vormalige Leitwert, gegebenenfalls auch automatisch selbstnachstellend, durch Verstellung wieder hergestellt werden.

Damit eignet sich das erfindungsgemässe System auch insbesondere für Einsätze, wo das Analysegas reaktive Anteile aufweist.

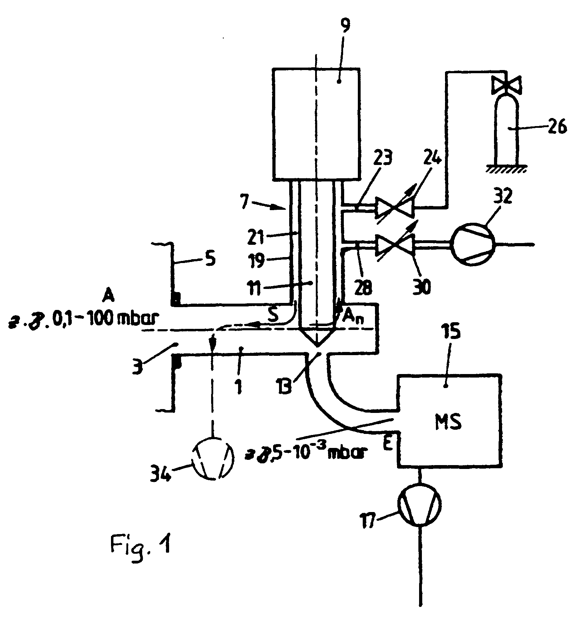

## Patentansprüche

1. Anordnung zum Verbinden eines Tiefdruckanschlusses (E) eines Gasanalysegerätes (15) mit einem auf höherem Druckniveau betriebenen Testgasanschluss (A), umfassend:
- eine Verbindungsleitung mit einem Anschluss für den Tiefdruckanschluss (E) sowie einem Anschluss für den Testgasanschluss (A);
- einen weiteren, in die Verbindungsleitung einmündenden Gasströmungsanschluss (21);
- einen in der Verbindungsleitung (1) wirkenden, deren Gasströmungsquerschnitt zum Anschluss an den Tiefdruckanschluss steuernden Ventilkörper (11),
**dadurch gekennzeichnet, dass** der Ventilkörper (11) innerhalb eines mit der Verbindungsleitung am weiteren Gasströmungsanschluss verbundenen Leitungsabschnittes (21) geführt ist und der Gasströmungsquerschnitt des Leitungsabschnittes (21) zwischen dessen Innenwand und dem Ventilkörper gebildet ist.

2. Anordnung nach Anspruch 1, **dadurch gekennzeichnet, dass** an den Leitungsabschnitt (21) eine Gasquelle (26) und/oder eine Vakuumpumpe (32) angeschlossen ist.

3. Anordnung nach einem der Ansprüche 1 oder 2, **dadurch gekennzeichnet, dass** zwischen einer mittels des Ventilkörpers. (11) gesteuerten Druckstufe in der Verbindungsleitung und dem Anschluss für den Testgasanschluss (A) ein Pumpenanschluss (34) in die Verbindungsleitung (1) einmündet.

4. Anordnung nach einem der Ansprüche 1 bis 3, **dadurch gekennzeichnet dass** der Strömungsquerschnitt einer durch den Ventilkörper in der Verbindungsleitung gebildeten Druckstufe mittels dem Ventilkörper gesteuert variierbar ist.

5. Massenspektrometer, vorzugsweise Quadrupolmassenspektrometer, mit einer Anordnung nach einem der Ansprüche 1 bis 4 am Tiefdruckanschluss.

6. Verwendung der Anordnung nach einem der Ansprüche 1 bis 4 bzw. eines Massenspektrometers nach Anspruch 5 für die Analyse von Gasen mit reaktivem Gasanteil.

## Claims

1. Arrangement for connecting a low pressure connection (E) of a gas analysis device (15) with a test gas connection (A) operated at a higher pressure level, comprising:
- a connecting line with a connection for the low pressure connection (E) and a connection for the test gas connection (A);
- a further gas flow connection (21) opening into the connecting line;
- a valve body (11) acting in the connecting line (1) and controlling its gas flow cross section to the connection for the low pressure connection,
**characterised in that** the valve body (11) is guided within a line section (21) connected with the connecting line at the further gas flow connection, and the gas flow cross section of the line section (21) is formed between its inner wall and the valve body.

2. Arrangement according to claim 1, **characterised in that** connected to the line section (21) is a gas source (26) and/or a vacuum pump (32).

3. Arrangement according to any of claims 1 or 2, **characterised in that** between a pressure stage controlled by means of the valve body (11) in the connecting line and the connection for the test gas connection (A), a pump connection (34) opens into the connecting line (1).

4. Arrangement according to any of claims 1 to 3, **characterised in that** the flow cross section of a pressure stage formed by the valve body in the connecting line is variable and controlled by means of the valve body.

5. Mass spectrometer, preferably quadrupole mass spectrometer, with an arrangement according to any of claims 1 to 4 at the low pressure connection.

6. Use of the arrangement according to any of claims 1 to 4 or of a mass spectrometer according to claim 5 for the analysis of gases with a reactive gas component.

## Revendications

1. Dispositif pour relier un raccordement basse pression (E) d'un appareil d'analyse de gaz (15) à un raccordement de gaz de test (A) fonctionnant à un niveau de pression plus élevé, comprenant :
- un conduit de liaison comportant un raccord pour le raccordement basse pression (E) et un raccord pour le raccordement de gaz de test (A) ;
- un autre raccordement d'écoulement de gaz (21) qui débouche dans le conduit de liaison ;
- un corps de soupape (11) qui agit dans le conduit de liaison (1) et qui commande la section transversale d'écoulement de gaz de celui-ci en vue du raccordement au raccordement basse pression,
**caractérisé en ce que** le corps de soupape (11) passe à l'intérieur d'un tronçon de conduit (21) relié au conduit de liaison au niveau de l'autre raccordement d'écoulement de gaz, et la section transversale d'écoulement de gaz dudit tronçon (21) est définie entre la paroi intérieure de celui-ci et le corps de soupape.

2. Dispositif selon la revendication 1, **caractérisé en ce qu'**une source de gaz (26) et/ou une pompe à vide (32) sont raccordées au tronçon de conduit (21).

3. Dispositif selon la revendication 1 ou 2, **caractérisé en ce qu'**entre un étage de pression commandé à l'aide du corps de soupape (11) dans le conduit de liaison, et le raccord pour le raccordement de gaz de test (A), un raccordement de pompe (34) débouche dans ledit conduit de liaison (1).

4. Dispositif selon l'une des revendications 1 à 3, **caractérisé en ce que** la section transversale d'écoulement d'un étage de pression défini par le corps de soupape dans le conduit de liaison est variable de manière commandée à l'aide dudit corps de soupape.

5. Spectromètre de masse, de préférence spectromètre de masse quadripolaire, comportant un dispositif selon l'une des revendications 1 à 4 au niveau du raccordement basse pression.

6. Utilisation du dispositif selon l'une des revendications 1 à 4 ou d'un spectromètre de masse selon la revendication 5 pour l'analyse de gaz contenant une proportion de gaz réactive.
